# EUROPEAN PATENT SPECIFICATION

(11) **EP 2 400 917 B1**
(45) Date of publication and mention of the grant of the patent: **04.12.2013**
(21) Application number: 10707455.1
(22) Date of filing: 25.02.2010
(51) Int. Cl.: A61C 8/00

(54) **DEVICE FOR INDICATING THE POSITION AND ORIENTATION OF A DENTAL IMPLANT**
VORRICHTUNG ZUR ANZEIGE DER POSITION UND ORIENTIERUNG EINES ZAHNIMPLANTATS
DISPOSITIF POUR INDIQUER LA POSITION ET L'ORIENTATION D'UN IMPLANT DENTAIRE

(30) Priority: 26.02.2009 US 155675 P
(43) Date of publication of application: 04.01.2012
(73) Proprietor: Nobel Biocare Services AG, 8058 Zürich-Flughafen (CH)
(72) Inventor: ERIKSSON, Thomas, S-414 60 Göteborg (SE); OTTSJÖ, Magnus, S-421 43 Västra Frölunda (SE)
(74) Representative: Roman Ekstedt, Måns
(86) International application number: PCT/EP2010/001153
(87) International publication number: WO 2010/097214

(56) References cited:
- EP-A1- 2 130 514
- EP-A2- 1 500 380
- WO-A1-96/16611
- WO-A1-2008/041943
- US-A- 6 126 445
- US-B1- 6 590 654

## Description

### Field of the Invention

This invention pertains in general to the field of oral, dental or maxillofacial restorative medical procedures, and products related thereto. More particularly the invention relates to a device and a method for facilitating determination of a position and orientation of components, like surgical implants, involved in such procedures. Even more particularly, the invention relates to position locators affixable to said components in a defined relationship, having at least one surface detectable by optical scanning for the position and orientation determination.

### Background of the Invention

An optical scanning method and apparatus is for instance disclosed in US 6,590,654.

WO-A-2008 041 943 describes a position locator device for a system of planning and producing oral or maxillofacial restorative products. This position locator device is made of titanium. Its outer surface is detectable by an optical scanner apparatus for determining the position and orientation of said outer surface. The outer surface is optically more diffusive reflective than specularly reflective. A paint layer based on titanium dioxide is deposited onto the outer surface of the position locator device. The body of the position locator device has a cylindrical portion, a top portion, a threaded portion and a connection interface for connection to a dental implant.

EP-A-1 500 380 describes a position locator for a system of planning and producing oral or maxillofacial restorative products. The body of the position locator device has a cylindrical portion, a conical portion, a top portion, a threaded portion and a connection interface for connection to a dental implant The conical portion and the top portion form a frusto conical portion which has a longitudinal center axis that coincides with the longitudinal center axis of the position locator device.

EP-A-2 130 514, which was published on 09.12.2009, describes a position locator device for a system of planning and producing oral or maxillofacial restorative products. This position locator device is made of plastic material. Its outer surface is detectable by an optical scanner apparatus for determining the position and orientation of said outer surface. The outer surface is optically more diffusive reflective than specularly reflective. A layer of poorly reflective material is deposited onto the outer surface of the position locator device. The body of the position locator device has a cylindrical portion, a conical portion, a top portion, and a connection interface for connection to a dental implant The conical portion and the top portion form a frusto conical portion which has a longitudinal center axis that coincides with the longitudinal center axis of the position locator device.

A surface treatment process for producing an oxide layer on a metallic object is disclosed in WO 00/72776 of the same applicant as the present application. The surface treatment process is described in WO 00/72776 with reference to implants for bone or tissue structures. An application of the method with position locators is not disclosed. Further, the purpose of the process described in WO 00/72776 is to provide a porous surface layer on an implant that has advantageous osseointegration properties.

There is a need for providing position locating devices for detection by such optical scanning apparatuses, which are advantageous from a point of view of detectability with high precision. These position locating devices are hereinafter also referred to as position locators.

An application where such position locators are needed is the field of dental restorative procedures.

### Summary of the Invention

Accordingly, embodiments of the present invention preferably seek to mitigate, alleviate or eliminate one or more deficiencies, disadvantages or issues in the art, such as the above-identified, singly or in any combination by providing a position locator device, a kit, and a method according to the appended claims.

According to a first aspect of the invention, a position locator device is provided. The position locator device is provided for a system of planning and producing oral or maxillofacial restorative products. The position locator device is made of an optically opaque metal and has at least one outer surface that is detectable by an optical scanner apparatus for determining at least one of the position and orientation of the outer surface. The outer surface is optically more diffusive reflective than specularly reflective. The device further has a metal oxide layer deposited onto the outer surface by anodic oxidation. The device further has a body with a cylindrical portion, a conical portion, a top portion, a threaded portion and a connection interface for connection to a dental implant or dental implant replica. The conical portion and the top portion form a frusto conical portion which has a longitudinal center axis that coincides with the longitudinal center axis of the position locator device.

In some embodiments, the metal oxide layer has a homogenous layer thickness.

In some embodiments, the metal is titanium.

In some embodiments, the outer surface is a porous titanium oxide layer deposited onto a body of the position locator device by anodic oxidation.

According to a second aspect of the invention, a kit of at least one implant replica or an abutment replica and at least one position locator according to the first aspect of the invention is provided.

According to another aspect of the invention, a method is provided. The method is a method of improving precision in an optical scanning method for determining the position of a dental implant. The method comprising providing a position locator according to the first aspect of the invention for connection by means of a helically threaded portion to the dental implant and performing the optical scanning method.

In some embodiments the optical scanning method is conoscopic holographic scanning.

Further embodiments of the invention are defined in the dependent claims.

Some embodiments of the invention provide for improved precision of determination of a position and orientation of a dental implant by optical scanning.

Some embodiments of the invention also provide for providing input data with improved precision for virtually planning a dental restoration, and thus production data for dental restorations is provideable with improved precision.

Some embodiments of the invention provide for an improved detectability of position locators by optical scanning in combination with improved precision of the dimensions of the position locators within desired tolerances for dental restorative procedures.

Some embodiments of the invention also provide for improved matching or merging of a plurality of data sets thanks to improved resolution of data provided by optical scanning of improved position locators.

Some embodiments provide for improved merging of patient data and data for a dental restoration.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a flow chart of a method;
Figs. 2A-E are various views of an embodiment of a position locator;
Figs. 3 and 4 are perspective views of a plurality of position locators affixed to a model of a patient's jaw; and
Figs. 5A-D are various views of another embodiment of a position locator.

### Description of embodiments

Specific embodiments of the invention will now be described with reference to the accompanying drawings.

The following description focuses on an embodiment of the present invention applicable to a dental restorative procedure. However, it will be appreciated that the invention is not limited to this application but may be applied to many other surgical procedures, including for example maxillofacial restorative surgical procedures.

### Position locators:

A position locator device for a system of planning and producing oral or maxillofacial restorative products is provided. The position locator device is made of an optically opaque metal, and has at least one outer surface detectable by an optical scanner apparatus for determining a position and orientation of said outer surface. In this manner , a position and orientation of the position locator device is determinable

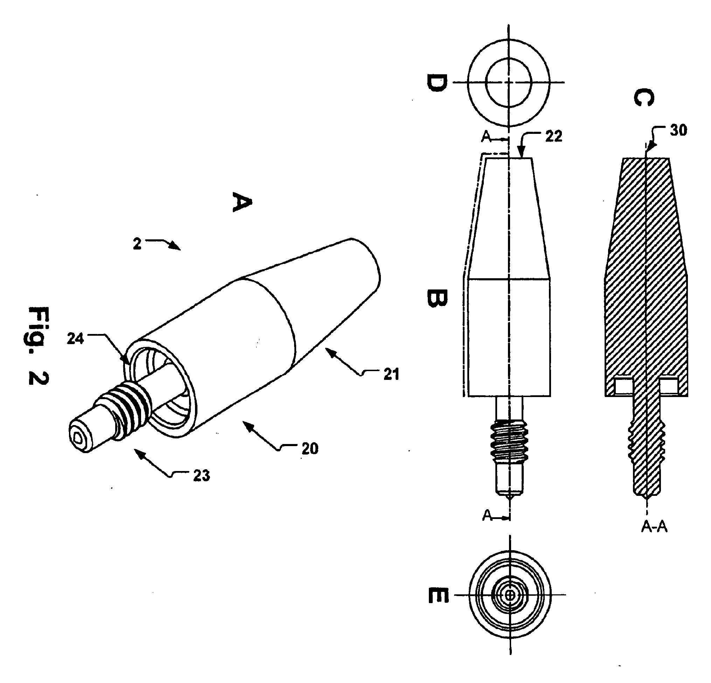

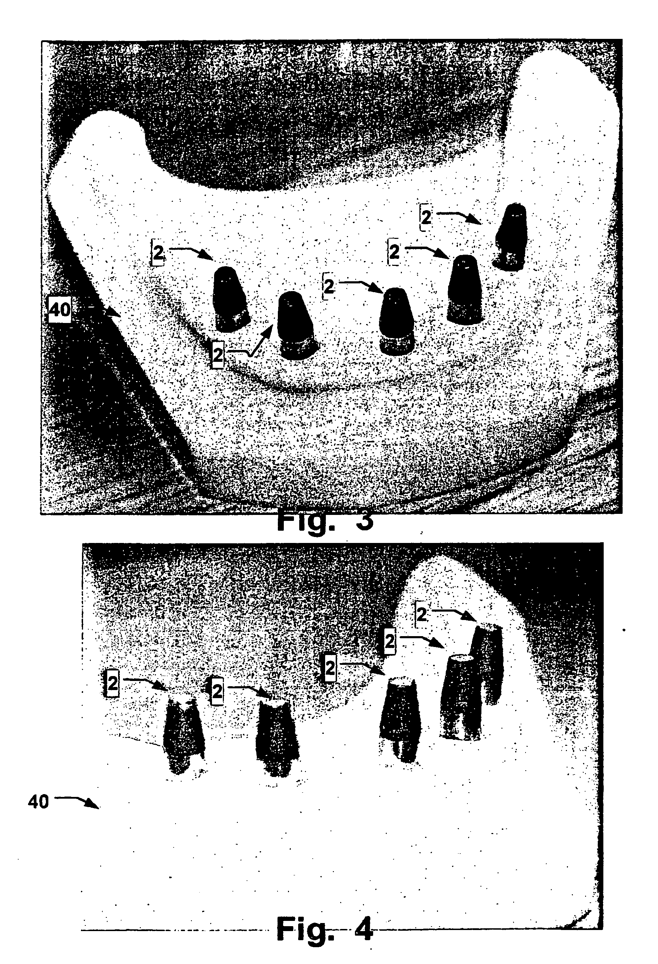

An embodiment of a position locator 2 is illustrated in Figs. 2A-E. In Figs. 3 and 4 such a position locators are illustrated inserted into dental implant replicas in a model 40 of a dental situation of a patient prior to providing a final dental restoration.

The position locator has a cylindrical portion 20, a conical portion 21, a top portion 22, a threaded portion 23 and a connection interface 24.

At least a portion of the position locator surface is dull for improved optical scanning and measurement of positions thereof, as is described further below. The diffuse reflection provided by the surface provides high precision when optically scanned.

Preferably, the position locator body is made of titanium. The surface is an oxidized surface of the metal. The oxidized surface has a layer thickness. The oxidized surface has a surface roughness. The geometrical dimensions of the position locator are provided with very high precision, as will be explained below.

The conical portion and the top portion provide a frusto conical portion which longitudinal center axis coincides with a longitudinal center axis 30 of the position locator device 2.

The conical portion 21 is used for calculation of the longitudinal center axis 30. The top surface 22 of the frusto conical portion is used to determine the position in space of the position locator 2. In this manner, a vector for the position and orientation of the position locator 2 is determinable by optical scanning. When the position locator 2 is affixed to another structure having a mating connection interface, such as a dental implant, it has a precisely defined geometrical relation and position in relation to the other structure. The position locator 2 is affixed to another structure by means of the helically threaded portion 23. By registering the position locator 2, the position of the other structure can be determined as there is a defined relation between the two.

The position locators may be rotationally symmetrical. Rotationally symmetry may be used in applications where the rotational orientation of the position locator is not needed or not of importance. This is for instance the case when the rotation of a dental implant around the longitudinal axis thereof is irrelevant. This is for instance the case when at least two implants are used to bear a connecting structure providing rotational locking itself. It is sufficient to use rotationally symmetrical position locators. For instance dental bridges are fixated to a jaw of a patient by using at least two dental implants. Here, the position and longitudinal orientation of the implants are determined by using rotationally symmetrical position locators, such as illustrated in Figs. 3 and 4.

Rotationally non-symmetrical are provided in some embodiments (not shown). Non-symmetry may be provided by non-symmetric top surfaces, or laterally plane surfaces e.g. in a conical portion.

The position locators may have different diameters.

The position locators may have different mating interfaces for connection interfaces of components to which they are releasably attachable.

Preferably, the position locator is made of titanium. Titanium is an advantageous material for a body of position locators because a titanium oxide layer is provideable on a surface thereon that is particularly optically advantageous, as is described below.

The position locators are for instance produced by turning and/or milling from a raw material. The manufacturing method applied depends on various factors, including the shaping of the position locator, the connection interface thereof, etc. Rotationally symmetrical position locators may be manufactured by turning only. Rotationally symmetrical position locators may be manufactured partly by turning a raw material into a rotationally symmetrical intermediate product. The intermediate product is subsequently machined to comprise at least one non-rotationally symmetrical surface.. This is for instance done by milling or cutting, whereby material is removed from the intermediate product. Thereby a surface is formed that has a defined relation to a rotational position in longitudinal direction of an implant when the position locator is affixed thereto. Such a position locator providing a rotational registration may be used for a dental implant for a single tooth, where the rotational locking capability of the implant is of importance.

By frequently used machining steps, such as milling or turning, of a metal, and in particular titanium, the machined surface of the positioning locator body becomes very smooth and glossy. Such machined surfaces have proven to be less well suited for detection by optical scanners, such as described in US 6,590,654. In particular, US 6,590,654 refers to conoscopic holographic scanning. Scanning position locators having machined surfaces results in erroneous measurements. Instable measurement values are obtained. This is due to the optical properties of the machined surfaces. The machined surfaces are too glossy. Incident light from the scanner light source is mostly reflected and does not reach the optical detector, which is oriented in the same direction as the incident light beam. When the incident light beam is directed non-perpendicularly onto a machined surface, it is reflected away. In this manner, a too low or no backscattered light reaches the detector and a position and orientation measurement cannot be provided correctly. For instance a position and orientation of a cylindrical, partly frusto conical position locator is not determined sufficiently well for providing data suitable for producing components to be used in dental restoration procedures. The measurement result has to lie within tolerances of as low as in the range of a few micrometers. This is in particular of importance, where a plurality of dental implants are used for affixing a single structure, like a dental bridge, to a jaw. In case the dental bridge framework is produced from erroneous data, it will not fit the implants installed in the patient. Also for single tooth restorations, such narrow tolerances are needed in order to provide dental restorations that fit in relation to surrounding teeth. In case a single tooth restoration is produced from erroneous data, it will not fit into the space provided by the surrounding teeth. This is costly and cumbersome for the patient and has to be avoided.

Thus, a finishing process to modify the machined surfaces to be less glossy is applied. The surface is made optically dull, such that diffuse scattering of incident light is predominant. However, as the position locator has to be provided with dimensions having very low deviation from a desired production value, this finishing process has to be carefully applied. It is desired that the finishing process as little as possible influences the physical dimension of the position locator.

Having in mind, that tolerances of as low as in the range of a few micrometers, are required, this is a demanding task. Many finishing procedures provide modified machined surfaces that are not suitable for this purpose. Finishing procedures may be based on removing material from the machined surface, or depositing layers of material onto the machined surface. However, most finishing procedures provide an inhomogeneous surface modification, which is not acceptable in the present case.

For instance abrasive blasting with e.g. sand is not suited for this purpose. Sandblasting is made with a jet stream of sand grains. This jet stream is inhomogeneous in itself due to the distribution of sand grains therein. Furthermore, overlapping treatment occurs by passing with the jet stream over the surface to be treated. Moreover, such sandblasting abrasion leads to a removal of more material than allowed within the presently desired tolerances. In this manner, it is impossible to control the finishing process to provide a homogenously finished surface within the desired tolerances.

Many techniques for creating layers having less glossy optical properties are not suited for the present purpose for similar reasons. For instance chemical vapor deposition (CVD) of a material onto a machined surface does not provide a sufficiently homogenous layer thickness. Variations of the layer thickness are larger than the allowed tolerances.

In this manner, although a sandblasted surface or a CVD treated surface has improved optical properties increasing the diffuse scattering, and thus may provide considerably better input signals to the optical scanning detector, the resulting measurements of such position locator would not be suited for providing data for production of dental restorative components. Such position locators provide input data being outside of the allowed tolerances. Hence, production data based on such input data and products manufactured on such production data are not suited for demanding applications such as dental restorative procedures and products.

A surface treatment process for producing an oxide layer on a metallic object is disclosed in WO 00/72776 of the same applicant as the present application.

In the context of the present application, the process described in WO 00/72776 is used in a novel manner. The process is used to provide improved optical properties of a surface. The process is applied on a position locator. In more detail, the process comprises anodic oxidation and is applied to position locators for providing an advantageously homogenous dull surface having improved precision of detectability by optical scanning.

The anodic oxidation process provides a very homogenous layer of oxide within very narrow tolerances. The process is advantageously controllable to provide a thin layer of oxide that lies within the desired tolerances, but considerably improves the optical properties of the surface with regard to optical scanning of the surface. In this manner, the final dimension of the position locator with the finished surface is determinable with high precision. The position locator is machined to a dimension which has the oxide layer thickness subtracted. When the oxide layer is added homogenously, the final product is provided to a desired dimension within very narrow tolerances, as required for dental restorative procedures and related products. Furthermore, the surface is advantageous for optical scanning, thus providing a very high precision of detection of a position and orientation of the position locator and related structures thereto.

Tests made by the applicant show that optical scanning of position locators having such modified surface provide a very good registration of the position and orientation of the position locator. Signals provided were very good, and considerably improved compared to position locators having machined surfaces.

The oxide layer is preferably made thinner than an oxide layer on implants. The layer thickness provided on position locators is in the range up to a few µm, such as up to 5 µm.

For providing the anodically oxidized surface, the position locator is positioned in an acid. Embodiments of specific acids are described in WO 00/72776. The anodic oxidation process is performed with a constant current and a voltage that is ramped up to a maximum voltage. The maximum voltage determines the layer thickness of the oxide layer and the process automatically ceases when the maximum voltage is obtained. The layer thickness obtained by the anodic oxidation process is thus controlled in a very precise manner. The maximum voltage is chosen lower than a maximum voltage that would be chosen for generating oxide layers on implants. A typical maximum voltage is in the range of 200-220 Volts.

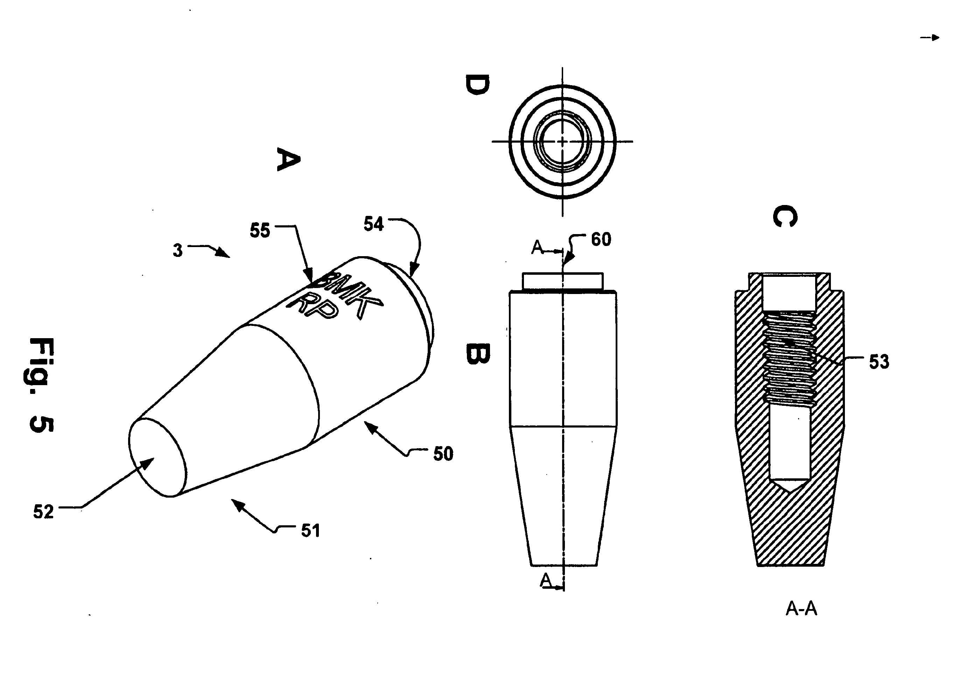

Furthermore, the anodic oxidation process allows for a controlled geometric coverage.of surfaces of position locators. The position locator may either only be submerged partly into acid during the anodic oxidation. Alternatively, or in addition, the position locator may be masked suitably to only allow oxidation of selected surfaces. The position locators may thus for instance be provided with surfaces that are not optically dull. The non treated surfaces may be provided with product markings, color coding, etc. such as illustrated in Fig. 5A at markings 55.

In another embodiment according to Figs. 5A-D, a position locator 3 is provided. The position locator 3 is a bridge position locator for attachment to a model of a dental restoration, like a model of a bridge framework. Thanks to the position locator, the position of the connection interface of a model of a dental restoration is determinable. This determined position of the connection interface is used in the method described below. The position locator 3 has a cylindrical portion 50, a conical portion 51, a top portion 52, an internally threaded portion 53 and a connection interface 54 for matingly engaging a dental restoration.

When an incident light ray is reflected from surfaces of optically opaque surfaces, the reflectance has several components, depending on the properties of the surface, such as roughness thereof. Three components of reflected light usually occur, namely a specular reflection, a specular lobe and a diffuse lobe.

For a metallic mirror surface, the incident light will be reflected by the surface following the law of reflection. In this case, the specular lobe is almost zero, the diffuse lobe is negligible, and the specular spike is very narrow and the intensity substantially corresponds to that of the incident light. In this case no backscattering occurs, and an optical detector arranged in direction of the incident ray will not detect a signal. Therefore, metallic mirror surfaces, like polished surface are not suitable for optical scanning.

For glossy or smooth surfaces, like the aforementioned machined metallic surfaces, the situation is similar. An incident light ray will be reflected by the surface following the law of reflection. In this case, the specular lobe is larger than in the case of a mirror surface, but directed mainly in the reflected direction; the diffuse lobe is larger, but negligible in the direction of the incident light and directed mainly in the reflected direction; and the specular spike is somewhat broader than in the previous case of mirror surfaces and has lost some. intensity to the aforementioned two lobes. Still, very little light is backscattered. Therefore, machined metallic surfaces, like turned or milled surfaces are not well suited for optical scanning.

For dull surfaces, like the aforementioned anodically oxidized surface, the situation is different. Here, the specular lobe is larger than in the case of the glossy or smooth surfaces, and much broader, deviating from the reflected direction; the diffuse lobe is largest and most intense, as well as a considerable portion of the incident light intensity is scattered back into the direction of the incident light; and the specular spike is almost non-existent or not at all existent. Therefore, the surface is optically more diffusive reflective than specularly reflective, and provides a portion of backscattered light that makes the dull surface well suited for detection by optical scanning with high precision.

The position locators of embodiments are provided with at least a surface of the latter category. The portion of light detectable from such surfaces when illuminated is improved considerably as the portion of the diffuse lobe in relation to the entire reflected light is dominant. This is in particular advantageous for optical scanning methods as described in US 6,590,654.

### Examples of use of position locators

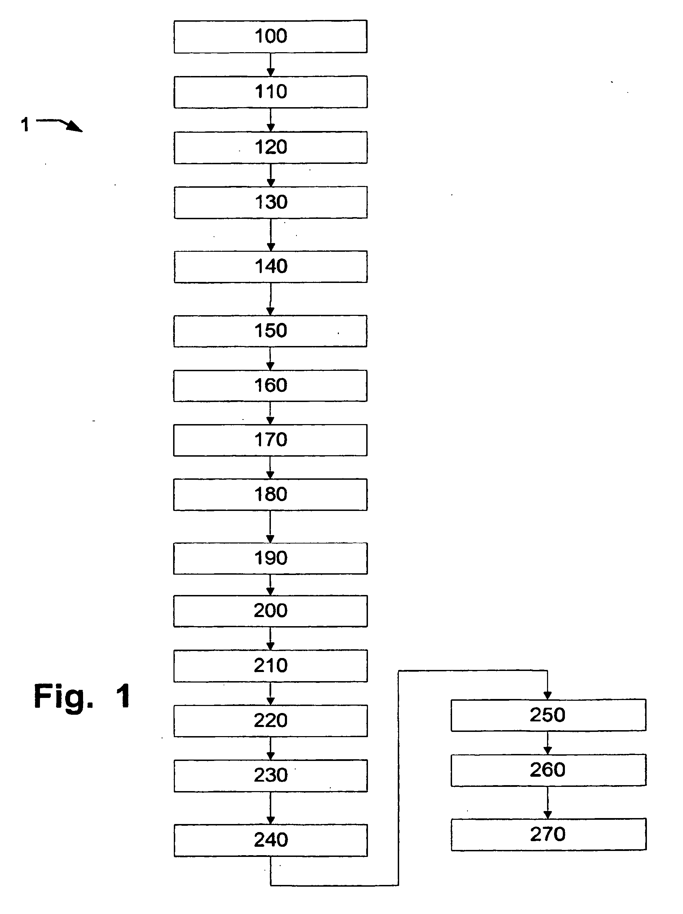

Fig. 1 is a flow chart of a method for preparing data and producing a dental restoration, using position locators during optical scanning.

In an initial point 100 of the method 1, an implant is provided implanted in bone tissue of a patient. After a healing and osseointegration period a component, such as a dental restoration, like a single tooth, a bridge, or other framework, have to be produced and affixed to the implant at a connection interface thereof. For this purpose, the exact position and orientation of the implant have to be determined.

To this end, an impression of the dental situation, is taken in step 120.

The position and orientation of an implant or an abutment in a model, or an impression coping in an impression has to be determined with high precision by optical scanning methods.

The impression is a negative of at least a portion of the oral cavity of a patient. The impression is made of a jaw portion comprising an already implanted dental implant. The implant has been implanted previously in the jaw bone tissue, and was optionally left in place for healing and osseointegration with the jaw bone tissue for a solid fixation therein. Thus the dental implant provides an anchored platform having a defined connection interface for fixation of dental restorations to the jaw bone. Dental restorations comprise dental bridges, single tooth restorations, etc. The dental restorations may be directly fixated to the dental implant or intermediate components like abutments may be used.

From the impression, data will be derived for the position and orientation of the dental implant. A plurality of dental implants may be present. As the implants are anchored in jaw bone tissue, only a top portion of the connection interface or abutment protruding into the oral cavity would be present in impressions. In order to provide improved orientation data for the longitudinal axis of the dental implant, impression copings and position locators are used during impression taking and subsequent scanning of the impression or a model prepared from the impression, respectively. This will now be explained in more detail.

The impression is taken from the patient with impression copings, which are attached to the dental implant(s) in step 110. This may be done by threadably insertion, snap fit, friction fit, or the like. The impression copings thus leave a negative impression in the impression, or are left in the impression when the impression material has solidified. The impression is then removed from the patient in step 130. As the impression copings are fixated to the dental implants, these are provided with a smooth surface such that they easily loosen from the solidified impression material. The impression copings are removed from the dental implants in step 140. The impression and the impression copings are then available for further processing steps towards producing a dental restoration.

An implant replica or an abutment replica is attached to the impression coping in step 150. This assembly is re-positioned in the impression into the corresponding recess in the impression in step 160. In this manner, the implant replica or the abutment replica protrudes from the impression at the exact position and in the precise orientation relative to the registered anatomical situation of the patient from which the impression was taken.

The position locator may also be used for intra oral scanning, wherein the position locator is attached directly to the implant in the oral cavity of the patient. In particular, the intra oral scanning is an optical scanning. The optical scanning is performed by means of an intra oral optical scanner.

A model is then cast from the impression in step 170. When the model is solidified, it is removed from the impression, together with the impression coping(s) in step 180. This model corresponds with high precision to the anatomical situation of the patient. Implant replica in the model are precisely positioned at correct positions and with correct orientation in the model. The impression coping(s) are then threadably removed from the fixated implant replica or abutment replica in step 190. As impression coping have a smooth surface these are not suited for optical scanning.

From the model, a dental restoration model, such as a bridge framework model is prepared in step 200, manually taking into consideration aesthetical, geometrical, and mechanical requirements. The bridge framework model may for instance be an acrylic framework prepared by using non-engaging temporary abutments or cylinders. The acrylic framework may then be manually reduced to a desired shape.

Subsequently, the model and the dental restoration model are scanned with an optical scanner to provide patient data and data for the dental restoration in steps 210 respectively 220. The patient data and data for the dental restoration are subsequently matched and provided in a virtual computer based environment in step 230 for planning the final restoration in step 240. Merging of the patient data and data for the dental restoration is facilitated thanks to the use of the improved position locators providing data with higher precision and resolution. When virtual planning is finished, production data is provided in step 250 for manufacturing of a dental restoration, such as a bridge framework, to be installed in the patient. The production is illustrated as step 260.

Prior to scanning, the position locators are threadably affixed to the implant replica or abutment replica. The position locators are now arranged in a precise relationship to the implant replica or abutment replica, corresponding to the anatomical situation of the patient. By detecting the position and orientation of the position locators from optical scan data, the exact position of the implant replica or abutment replica is determinable. This is very important for being able to provide high precision production data and finally dental restorations based on this production data.

Prior to scanning, position locators are also threadably affixed to the dental restoration model. These position locators provide, when scanned in an optical scanner, exact data for the position of a connection interface mating with a connection interface of the replica implants or replica abutments.

A plurality of dental implants or abutments may be present. In this case, one position locator will be used for every dental implant.

From optical scanning, the model and the dental restoration model are digitized to provide corresponding data. A calculation is made where the implants or copings are located. As the position locators provide high precision data from optical scanning, this is facilitated by the position locators. For instance, the conical portion of the frusto conical portion is used for calculation of the longitudinal center axis of a position locator. The top surface of the frusto conical portion of the position locator is used to determine the position in space of the position locator. In this manner, a vector for the position and orientation of the position locator is determined. As the longitudinal axis of the position locator and the dental implant or abutment coincide or have a defined relationship, the orientation of the dental implant or abutment is determinable with high precision. As the top portion is provided with high precision and has a defined relation to the connection interface, also the exact position of the dental implant or abutment is determinable.

Also, the dental restoration model having position locator(s) affixed thereto provides data for the position of the connection interface mating with the connection interface of the dental implants or abutments with high precision. Position locators for the dental restoration model, which for instance are shown in Figs. 5A-D, differ from position locators for implants or abutments, which for instance are shown in Figs. 2A-E, with regard to the connection interface. The connection interfaces of the dental restoration model and the implants or abutments are mating male/female connection interfaces. Consequently, the connection interfaces are matingly arranged female/male connection interfaces.

The connection interfaces themselves differ in dependence of the specific implant system used.

The patient data and data for the dental restoration are subsequently matched and provided in a virtual computer based environment for planning the final restoration.

When virtual planning is finished, production data is provided for manufacturing of a dental restoration, such as a bridge framework, to be installed in the patient. Further adaptation of the manufactured product, e.g. veneering may be performed manually before finally installing the dental restoration in the patient. Installation is performed smoothly in step 270 as the dental restoration perfectly fits to the dental implants already installed in the patient.

The present invention has been described above with reference to specific embodiments. The scope of the invention is only limited by the appended claims.

## Claims

1. A position locator device (2,3) for a system of planning and producing oral or maxillofacial restorative products, said device being of an optically opaque metal, and having:
at least one outer surface detectable by an optical scanner apparatus for determining at least one of a position and orientation of said outer surface, said outer surface being optically more diffusive reflective than specularly reflective,
a metal oxide layer deposited onto said outer surface of said position locator device by anodic oxidation,
a body having a cylindrical portion (20), a conical portion (21), a top portion (22), a threaded portion (23) and a connection interface (24) for connection to a dental implant or dental implant replica, said conical portion (21) and said top portion (22) forming a frusto conical portion which has a longitudinal center axis that coincides with a longitudinal center axis of said device.

2. The device according to claim 1, wherein said metal oxide layer has a substantially homogenous layer thickness.

3. The device according to claim 1, wherein said metal is titanium.

4. The device according to claim 3, wherein said outer surface is a porous titanium oxide layer deposited onto a body of said position locator device by anodic oxidation.

5. The device according to any of claims 1-4, wherein said device comprises a body being at least partly rotationally symmetrical.

6. The device according to any of claims 1-5, wherein said device comprises a body having a rotationally non-symmetrical portion related to a defined rotational position in longitudinal direction of an implant to which said device is attachable.

7. The device according to any of claims 1 to 6, wherein said outer surface is an outer surface of said conical portion (21) and said top portion (22).

8. The device according to any of claims 1 to 7, wherein said connection interface (24) is a connection interface mating with a connection interface of an implant to which said device is attachable for determining a position and orientation thereof.

9. A kit of at least one implant replica or abutment replica and at least one position locator according to any of claims 1 to 8.

10. A method of improving precision in an optical scanning method for determining the position of a dental implant, comprising providing a position locator according to any of claims 1 to 8 for connection by means of a helically threaded portion (23) to said dental implant and performing said optical scanning method.

11. The method according to claim 10, wherein said optical scanning method is conoscopic holographic scanning.

## Patentansprüche

1. Positionsgebervorrichtung (2, 3) für ein System zur Planung und Herstellung von restaurativen Produkten für Mund oder Gesicht, wobei die Vorrichtung aus einem optisch undurchlässigen Metall besteht und Folgendes aufweist:
zumindest eine äußere Oberfläche, die durch eine optische Abtastvorrichtung detektierbar ist, um zumindest eine Position oder Orientierung der äußeren Oberfläche zu bestimmen, wobei die äußere Oberfläche optisch mehr diffus reflektierend als spiegelnd reflektierend ist,
eine Metalloxidschicht, die auf der äußeren Oberfläche der Positionsgebervorrichtung durch anodische Oxidation abgeschieden wird,
einen Körper mit einem zylindrischen Teil (20), einem konischen Teil (21), einem oberen Teil (22), einem Gewindeteil (23) und einer Verbindungsschnittstelle (24) zur Verbindung mit einem Zahnimplantat oder einer Replik eines Zahnimplantats, wobei der konische Teil (21) und der obere Teil (22) einen kegelstumpfförmigen Teil bilden, der eine Mittelachse in Längsrichtung aufweist, die mit einer Mittelachse der Vorrichtung in Längsrichtung übereinstimmt.

2. Vorrichtung gemäß Anspruch 1, wobei die Metalloxidschicht eine im Wesentlichen homogene Schichtdicke aufweist.

3. Vorrichtung gemäß Anspruch 1, wobei das Metall Titan ist.

4. Vorrichtung gemäß Anspruch 3, wobei die äußere Oberfläche eine poröse Titanoxidschicht ist, die auf einen Körper der Positionsgebervorrichtung durch anodische Oxidation abgeschieden wird.

5. Vorrichtung gemäß einem der Ansprüche 1-4, wobei die Vorrichtung einen Körper umfasst, der zumindest zum Teil rotationssymmetrisch ist.

6. Vorrichtung gemäß einem der Ansprüche 1-5, wobei die Vorrichtung einen Körper umfasst, der einen nicht-rotationssymmetrischen Teil, bezogen auf eine definierte Drehposition in Längsrichtung eines Implantats, an das die Vorrichtung anbringbar ist, aufweist.

7. Vorrichtung gemäß einem der Ansprüche 1 bis 6, wobei die äußere Oberfläche eine äußere Oberfläche des konischen Teils (21) und des oberen Teils (22) ist.

8. Vorrichtung gemäß einem der Ansprüche 1 bis 7, wobei die Verbindungsschnittstelle (24) eine Verbindungsschnittstelle ist, die mit einer Verbindungsschnittstelle eines Implantats, an das die Vorrichtung anbringbar ist, um eine Position und Orientierung davon zu bestimmen, ineinander greift.

9. Set aus zumindest einer Replik eines Implantats oder einer Replik eines Auflagers und zumindest einem Positionsgeber gemäß einem der Ansprüche 1 bis 8.

10. Verfahren zur Präzisionsverbesserung in einer optischen Abtastmethode zur Bestimmung der Position eines Zahnimplantats, das ein Vorsehen eines Positionsgebers gemäß einem der Ansprüche 1 bis 8 zur Befestigung an dem Zahnimplantat durch ein spiralförmiges Gewindeteil (23) und ein Ausführen der optischen Abtastmethode umfasst.

11. Verfahren gemäß Anspruch 10, wobei die optische Abtastmethode konoskopisch holographisches Abtasten ist.

## Revendications

1. Dispositif de localisation de position (2, 3) pour un système de planification et de production de produits de restauration orale ou maxillo-faciale, ledit dispositif étant formé d'un métal optiquement opaque, et ayant :
au moins une surface externe pouvant être détectée par un appareil de balayage optique pour déterminer au moins l'une d'une position et d'une orientation de ladite surface externe, ladite surface externe étant optiquement plus à réflexion diffusive qu'à réflexion spéculaire,
une couche d'oxyde métallique déposée sur ladite surface externe dudit dispositif de localisation de position par oxydation anodique,
un corps ayant une partie cylindrique (20), une partie conique (21), une partie supérieure (22), une partie filetée (23) et une interface de connexion (24) pour connexion à un implant dentaire ou à une réplique d'implant dentaire, ladite partie conique (21) et ladite partie supérieure (22) formant une partie tronconique qui a un axe central longitudinal qui coïncide avec un axe central longitudinal dudit dispositif.

2. Dispositif selon la revendication 1, dans lequel ladite couche d'oxyde métallique a une épaisseur de couche essentiellement homogène.

3. Dispositif selon la revendication 1, dans lequel ledit métal est le titane.

4. Dispositif selon la revendication 3, dans lequel ladite surface externe est une couche poreuse d'oxyde de titane déposée sur un corps dudit dispositif de localisation de position par oxydation anodique.

5. Dispositif selon l'une des revendications 1-4, dans lequel ledit dispositif comprend un corps qui est au moins partiellement symétrique en rotation.

6. Dispositif selon l'une des revendications 1-5, dans lequel ledit dispositif comprend un corps ayant une partie non symétrique en rotation relative à une position de rotation définie dans une direction longitudinale d'un implant auquel ledit dispositif peut être fixé.

7. Dispositif selon l'une des revendications 1 à 6, dans lequel ladite surface externe est une surface externe de ladite partie conique (21) et de ladite partie supérieure (22).

8. Dispositif selon l'une des revendications 1 à 7, dans lequel ladite interface de connexion (24) est une interface de connexion accouplée à une interface de connexion d'un implant auquel ledit dispositif peut être fixé pour déterminer une position et une orientation de celui-ci.

9. Kit d'au moins une réplique d'implant ou une réplique de pilier et d'au moins un localisateur de position selon l'une des revendications 1 à 8.

10. Procédé d'amélioration de précision dans un procédé de balayage optique pour déterminer la position d'un implant dentaire, comprenant le fait de fournir un localisateur de position selon l'une des revendications 1 à 8 pour connexion audit implant dentaire au moyen d'une partie filetée en hélice (23) et d'effectuer ledit procédé de balayage optique.

11. Procédé selon la revendication 10, dans lequel ledit procédé de balayage optique est un balayage holographique conoscopique.
